# EUROPEAN PATENT SPECIFICATION

(11) **EP 0 188 316 B1**
(45) Date of publication and mention of the grant of the patent: **27.10.1993**
(21) Application number: 86300054.3
(22) Date of filing: 07.01.1986
(51) Int. Cl.: C12P 13/02, C12N 9/78

(54) **Process for the preparation of amides using microorganisms**
Verfahren zur Herstellung von Amiden unter Verwendung von Mikroorganismen
Procédé de préparation d'amides utilisant des microorganismes

(30) Priority: 08.01.1985 JP 452/85
(43) Date of publication of application: 23.07.1986
(73) Proprietor: NITTO CHEMICAL INDUSTRY CO., LTD., Tokyo (JP)
(72) Inventor: Watanabe, Ichiro, Yokosuka-shi Kanagawa (JP); Okumura, Masami, Yokohama-shi Kanagawa (JP)
(74) Representative: Pearce, Anthony Richmond

(56) References cited:
- EP-A- 0 178 106
- EP-A- 0 204 555
- GB-A- 2 018 240
- US-A- 4 001 081
- US-A- 4 629 700
- AGRIC. BIOL. CHEM., vol. 46, no. 5, 1982, pages 1165-1174; Y. ASANO et al.:"Aliphatic nitrile hydratase from Arthrobacter sp. J-1 purification and characterization"
- CHEMICAL ABSTRACTS, vol. 101, no. 24, 10th December 1984, page 274, abstract no.215828r, Columbus, Ohio, US; H. YANG et al.: "A new kind of mixed bacterial culture degrading sodium thiocyanate and acrylonitrile"
- CHEMICAL ABSTRACTS, vol. 98, no. 5, 31st January 1983, page 370, abstract no.31108u, Columbus, Ohio, US; J.M. MILLER et al.: "The utilization of nitrilesandamides by a Rhodococcus species"

## Description

### FIELD OF THE INVENTION

The present invention relates to a process for the preparation of amides using microorganisms. More particularly, it is concerned with a process for hydrating nitriles by the action of microorganisms, to thereby prepare the corresponding amides.

### BACKGROUND OF THE INVENTION

In recent years, extensive investigations have been increasingly made on utilization of microorganisms and enzymes as catalysts for various productions of chemical substances.

An enzyme capable of hydrating nitriles to form the corresponding amides is known as nitrilase or nitrilehydratase. It has been described that bacteria belonging to the genus Bacillus, the genus Bacteridium in the sense of Prevot, the genus Micrococcus and the genus Brevibacterium (Japanese patent application (OPI) No. 86186/76 (corresponding to U.S. Patent 4,001,081) (the term "OPI" as used herein refers to a "published unexamined Japanese patent application")), bacteria belonging to the genus Corynebacterium and the genus Norcardia (Japanese Patent Publication No. 17918/81, corresponding to U.S. Patent 4,248,968), and bacteria belonging to the genus Pseudomonas (Japanese Patent Publication No. 37951/84) have nitrilase activity and hydrate nitriles to form the corresponding amides, particularly acrylonitrile, to form acrylamide.

### SUMMARY OF THE INVENTION

The present invention is directed to a process for preparing an amide compound using microorganisms, which comprises subjecting a mononitrile having from 2 to 6 carbon atoms to the action of a bacterium having an ability to hydrate the mononitrile to form the corresponding amide in an aqueous medium characterised in that the bacterium belongs to the genus Rhodococcus or the genus Microbacterium, but with the proviso that the use of strain B222 (CBS 498.74) is excluded.

### DETAILED DESCRIPTION OF THE INVENTION

The process of the present invention is particularly effective in the preparation of acrylamide from acrylonitrile.

The microorganisms used according to the present invention are bacteria having nitrilase activity belonging to the genus Rhodococcus and the genus Microbacterium. Typical examples are Rhodococcus sp. S-6 FERM BP-687, Rhodococcus erythropolis IFM 155, Rhodococcus rhodochrous IFM 153, and Microbacterium flavum IAM 1642.

Bacteria designated by the symbols IFM, IFO, and IAM are known microorganisms and are readily available through The Japanese Federation of Culture Collections of Microorganisms (JFCC) of Research Institute for Chemobiodynamics, Chiba University (IFM); The Institute for Fermentation, Osaka (IFO); and Institute of Applied Microbiology, University of Tokyo (IAM), respectively. Rhodococcus sp. S-6 is a strain isolated by the present inventors, having particularly high nitrilase activity, and has been deposited in Fermentation Research Institute, Agency of Industrial Science & Technology, Ministry of International Trade and Industry, Japan, as FERM-BP No. 687. Bacteriological characteristics of the strain are shown below.

### Rhodococcus sp. S-6

(a) Morphology
   (1) Small rod-shaped, 0.5-0.8 µm diameter × 1-5 µm length
   (2) At an initial stage of cultivation, the cell is in a long rod-shaped form and irregular branches are observed. Later it breaks and splits into a spherical or short rod-shaped form (pleomorphism).
   (3) Motility: none
   (4) Formation of spore: none
   (5) Gram staining: positive (+)
   (6) Acid fastness: negative (-)
(b) Growth state in various culture media (30°C)
   (1) Bouillon agar Plate culture: colonies are circular, irregular, smooth in surface, and colored slightly pink.
   (2) Bouillon agar slant culture: good growth, trapezoidal in cross section, no luster, and slightly pink.
   (3) Bouillon liquid culture: vigorous growth while forming a pellicle, and the liquid is transparent and precipitates with growth.
(c) Physiological characteristics
   (1) Reduction of nitrate: positive (+)
   (2) Decomposition of urea: Positive (+)
   (3) Indole production: negative (-)
   (4) Hydrolysis of starch: negative (-)
   (5) Decomposition of gelatin: negative (-)
   (6) Decomposition of cellulose: negative (-)
   (7) Oxidase: negative (-)
   (8) Catalase: positive (+)
   (9) Requirement of a free oxygen: positive (+)
   (10) Growth in anaerobic condition: negative (-)
   (11) O/F test: 0
   (12) Growth at 37°C: positive (+)
   (13) Requirement of vitamins: negative (-)
   (14) Production of gas from glucose: negative (-)
   (15) Production of acid from glucose: Positive (+)
(d) Chemical composition of cells
   (1) Contains meso-diaminopimelic acid, arabinose and galactose (B. Becker et al., Applied Microbiology, Vol. 12, p. 421 (1964), and H.A. Lechevalier et al., The Actinomvcetales, p. 311 (1970)).
   (2) Contains fatty acids of C₁₆ (n, F₁), C₁₈ (F₁) and C₁₉ (10-CH₃) as main fatty acids (K. Komagata et al., International Journals of Systematic Bacteriology, Vol. 33 (2), p. 188 (1983)).
   (3) Contains C₃₂-C₄₆ mycolic acids as the mycolic acid type (M. Goodfellow, Microbiological Classification and Identification, (1980)).

Referring to Bergy's Manual of Determinative Bacteriology, H. Ans-G. Schlegel, The Prokaryotes, Vol. II (1981), anti the literature described in (d) concerning chemical classification of microorganisms, the strain S-6 is determined that it is a bacillus which is gram-positive, negative in formation of spore, aerobic, has polymorpholis, and is negative in acid fastness. This strain contains therein meso-diaminopimelic acid, arabinose and galactose, C₁₆ (n, F₁), C₁₈ (F₁) and C₁₉ (10-CH₃) as fatty acid types of acids, and C₃₂-C₄₆ mycolic acids as the mycolic acid type.

Based on the above bacteriological characteristics, the present strain is identified as a bacterium belonging to the genus Rhodococcus.

In cultivation of microorganisms as used herein, an ordinary culture medium containing a carbon source (e.g., glucose, glycerol, and maltose), a nitrogen source (e.g., ammonium sulfate and ammonium chloride), an organic nutrient source (e.g., yeast extract, peptone, meat extract, soybean protein hydrolyzate, and corn steep liquor (CSL)), an inorganic nutrient source (e.g., phosphate, magnesium, potassium, zinc, iron, and manganese), and so forth is used. This cultivation is aerobically carried out with stirring at a pH value of from 6 to 8 and at a temperature of from 20 to 35°C, and preferably 25 to 30°C, for from 1 to 3 days.

In the practice of the process of the present invention, one strain selected from the above microorganisms is cultured for 2 to 3 days according to the above-described method, and the resulting cultures or cells separated from the cultures, or treated cells (crude enzymes, immobilized cells, etc.) are suspended in water, a buffer or physiological saline water and then the mononitrile is added thereto.

The mononitrile is acted on the cells by reacting an aqueous medium generally containing from about 0.01 to 10 wt% of the cells and from about 0.1 to 10 wt% of the mononitrile at a temperature of from the freezing point thereof to 30°C, and preferably the freezing point to 15°C, at a pH of from 6 to 10, and preferably from 7 to 9, for a period of from 0.5 to 10 hours.

Mononitriles used as a substrate are biologically very toxic, and exert serious adverse influences on the present enzymatic reaction. For this reason, the mononitrile is gradually added in a controlled manner such that the concentration of mononitrile in the system is preferably not more than 5 wt%, and more preferably not more than 2 wt%.

If the pH value is outside the above-defined range, the amide formed and accumulated is further hydrolyzed, and the stability of the cells is reduced. Thus, it is preferred to control the pH value within the range of from 7 to 9 by gradually adding caustic alkali (e.g., NaOH and KOH), or by previously adding a buffer to the system.

If reaction conditions are appropriately controlled, the desired amide can be formed and accumulated from the mononitrile at a conversion value of nearly 100%, and with substantially no formation of by-products.

The amide thus formed can be recovered from the reaction mixture by commonly known techniques. For example, cells are separated from the reaction mixture by techniques such as centrifugal separation, treated with activated carbon, an ion exchange resin or the like to remove colored substances, impurities and the like, and then concentrated under reduced pressure to yield the desired amide, for example, acrylamide.

The present invention is described in greater detail with reference to the following examples. All parts and percents are by weight.

The various mononitriles and their corresponding amides were quantitatively analyzed by gas chromatography, and their corresponding organic acids by high performance liquid chromatography.

### EXAMPLE 1

A strain, Rhodococcus sp. S-6, was aerobically cultured on a medium (pH: 7.2) containing 1% of glucose, 0.5% of peptone, 0.3% of yeast extract, and 0.3% of meat extract at 30°C for 48 hours. The cells thus formed were removed by centrifugal separation and washed with a 0.05 M phosphate buffer (pH: 7.7). This procedure was repeated to prepare washed cells of the S-6 strain (water content: 80%).

A mixture of 0.5 part of the washed cells and 84.5 parts of a 0.05 M phosphate buffer (pH: 8.5) was prepared, and then 15 parts of acrylonitrile was intermittently added with stirring at from 0 to 3°C while controlling conditions such that the concentration of acrylonitrile in the reaction system did not exceed 2%, to thereby subject the acrylonitrile to a hydration reaction. Addition of acrylonitrile was completed in about 3 hours. To ensure the completion of the reaction, stirring was further continued for several hours. Then, cells were removed by centrifugal separation to yield a clear solution. This solution contained 20% of acrylamide, and the yield of acrylamide was more than 99.9%. Unreacted acrylonitrile was not detected at all, and the proportion of by-produced acrylic acid was not more than 0.1% (based on the weight of the acrylamide).

Water was distilled off from the clear solution at a temperature of not more than 50°C; the clear solution was concentrated to precipitate crystals. These crystals were recrystallized from methanol to yield colorless plate-shaped crystals. This compound was identified as acrylamide based on melting point, elementary analysis, and IR.

### EXAMPLE 2

Washed cells of the S-6 strain were obtained in the same manner as in Example 1 and measured for their reactivity to various mononitriles under the following conditions.
(a) Reaction Conditions

| | |
|---|---|
| Mononitrile | 2.5% |
| Potassium Phosphate Buffer | pH 7.7/0.05 M |
| Cells (as dry cells) | 5 mg |
| Temperature | 10°C |
| Reaction Time | 10 min |
| Amount of the Reaction Solution | 10 mℓ |

(b) Reaction Results

| Type of Mononitrile | Amide-Forming Activity* |
|---|---|
| Acetonitrile | 30 |
| Propionitrile | 102 |
| Acrylonitrile | 100 |
| Methacrylonitrile | 123 |
| Butyronitrile | 51 |
| Valeronitrile | 11 |
| Nicotinonitrile | 16 |

| | |
|---|---|
| * Relative value indicated with the activity to acrylonitrile as 100. | |

### EXAMPLE 3

100 mℓ of a culture medium comprising 1% of glycerol, 0.1% of KH₂PO₄, 0.05% of MgSO₄·7H₂O, 0.001% of FeSO₄·7H₂0, 0.5% of soybean protein hydrolyzate, and 0.1% of yeast extract (pH: 7.5) which had been sterilized and to which 0.5% of sterile isobutyronitrile had been added was prepared in 500 mℓ of an Erlenmeyer flask. Then, 1 mℓ of a culture of a type culture strain as shown below which had been cultured for 48 hours in the same culture medium as in Example 1 was added, and cultured with vibration at 25°C for 48 hours. After the cultivation was completed, cells were recovered by centrifugal separation and then washed with a 0.05 M phosphate buffer (pH: 7.7). This procedure was repeated to yield washed cells. These cells were measured for activity of formation of acrylamide from acrylonitrile in the same manner as in Example 2.

The results are shown in Table 1.

**TABLE 1**

| Type of Strain | Acrylamide-Forming Activity (µM/mg·hr) |
|---|---|
| Rhodococcus erythropolis IFM 155 | 3.5 |
| Rhodococcus rhodochrous IFM 153 | 2.5 |
| Microbacterium flavum IAM 1642 | 2.0 |

While the invention has been described in detail and with reference to specific embodiments thereof, it will be apparent to one skilled in the art that various changes and modifications can be made therein without departing from the scope thereof.

## Claims

1. A process for producing an amide compound utilizing microorganisms, which comprises subjecting a mononitrile having from 2 to 6 carbon atoms to the action of a bacterium having an ability to hydrate the mononitrile to form the corresponding amide in an aqueous medium, characterized in that said bacterium belongs to the genus Rhodococcus or the genus Microbacterium, but with the proviso that the use of strain B222 (CBS 498.74) is excluded.

2. A process as in claim 1, wherein the bacteria are used in the form of cultures, cells or treated cells.

3. A process as in claim 1, wherein the aqueous medium is maintained at a temperature of from the freezing point thereof to 30°C.

4. A process as in claim 1, wherein the aqueous medium is maintained at a temperature of from the freezing point thereof to 15°C.

5. A process as in claim 1, wherein the aqueous medium is maintained at a pH of from 6 to 10.

6. A process as in claim 1, wherein the aqueous medium is maintained at a pH of from 7 to 9.

7. A process as in claim 1, wherein the mononitrile is continuously or intermittently added to the aqueous medium such that the concentration of mononitrile is not more than 5 wt%.

8. A process as in claim 1, wherein the mononitrile is continuously or intermittently added to the aqueous medium such that the concentration of mononitrile is not more that 2 wt%.

9. A process as in claim 1, wherein the mononitrile is acrylonitrile, and the microorganism is Rhodococcus sp. S-6 (FERM-BP No. 687).

## Patentansprüche

1. Verfahren zur Herstellung einer Amidverbindung unter Verwendung von Mikroorganismen, umfassend das Unterwerfen eines Mononitrils mit 2 bis 6 Kohlenstoffatomen der Wirkung eines Bakteriums mit einer Fähigkeit, das Mononitril zur Bildung des entsprechenden Amids in einem wässrigen Medium zu hydratisieren,
dadurch **gekennzeichnet**, daß
das Baketrium dem Stamm Rhodococcus oder dem Stamm Microbacterium angehört, aber mit dem Vorbehalt, daß die Verwendung des Stammes B222 (CBS 498.74) ausgeschlossen ist.

2. Verfahren nach Anspruch 1,
dadurch **gekennzeichnet**, daß
die Bakterien in der Form von Kulturen, Zellen oder behandelten Zellen verwendet werden.

3. Verfahren nach Anspruch 1,
dadurch **gekennzeichnet**, daß
das wässrige Medium bei einer Temperatur von dem Gefrierpunkt davon bis zu 30°C gehalten wird.

4. Verfahren nach Anspruch 1,
dadurch **gekennzeichnet**, daß
das Medium bei einer Temperatur von dem Gefrierpunkt davon bis 15°C gehalten wird.

5. Verfahren nach Anspruch 1,
dadurch **gekennzeichnet**, daß
das wässrige Medium bei einem pH Wert von 6 bis 10 gehalten wird.

6. Verfahren nach Anspruch 1,
dadurch **gekennzeichnet**, daß
das wässrige Medium bei einem pH von 7 bis 9 gehalten wird.

7. Verfahren nach Anspruch 1,
dadurch **gekennzeichnet**, daß
das Mononitril kontinuierlich oder periodisch zu dem wässrigen Medium so zugegeben wird, daß die Konzentration des Mononitrils nicht mehr als 5 Gew.% ausmacht.

8. Verfahren nach Anspruch 1,
dadurch **gekennzeichnet**, daß
das Mononitril kontinuierlich oder periodisch zu dem wässrigen Medium so zugegeben wird, daß die Konzentration an dem Mononitril nicht mehr als 2 Gew.% ist.

9. Verfahren nach Anspruch 1,
dadurch **gekennzeichnet**, daß
das Mononitril Acrylnitril ist und daß der Mikroorganismus Rhodococcus sp. S-6 (FERM-BP Nr. 687) ist.

## Revendications

1. Procédé de production d'un amide à l'aide de microorganismes, dans lequel, dans lequel on soumet un mononitrile ayant 2 à 6 atomes de carbone à l'action d'une bactérie ayant la capacité d'hydrater le mononitrile pour former l'amide correspondant dans un milieu aqueux, caractérisé en ce que ladite bactérie appartient au genre Rhodococcus ou au genre Microbacterium, mais à condition que soit exclue l'utilisation de la souche B222 (CBS 498.74).

2. Procédé selon la revendication 1, dans lequel les bactéries sont utilisées sous forme de cultures, de cellules ou de cellules traitées.

3. Procédé selon la revendication 1, dans lequel le milieu aqueux est maintenu à une température comprise entre son point de congélation et 30°C.

4. Procédé selon la revendication 1, dans lequel le milieu aqueux est maintenu à une température comprise entre son point de congélation et 15°C.

5. Procédé selon la revendication 1, dans lequel le milieu aqueux est maintenu à un pH compris entre 6 et 10.

6. Procédé selon la revendication 1, dans lequel le milieu aqueux est maintenu à un pH compris entre 7 et 9.

7. Procédé selon la revendication 1, dans lequel le mononitrile est ajouté de façon continue ou intermittente au milieu aqueux de façon que la concentration en mononitrile ne soit pas supérieure à 5 % en masse.

8. Procédé selon la revendication 1, dans lequel le mononitrile est ajouté de façon continue ou intermittente au milieu aqueux de façon que la concentration en mononitrile ne soit pas supérieure à 2 % en masse.

9. Procédé selon la revendication 1, dans lequel le mononitrile est l'acrylonitrile et le microorganisme est Rhodococcus sp. S-6(FERM-BP n° 687).
